(19) Europäisches Patentamt
European Patent Office
Office européen des brevets

(11) Publication number: **0 342 422 A2**

# (12) EUROPEAN PATENT APPLICATION

(21) Application number: 89107839.6

(22) Date of filing: 29.04.89

(51) Int. Cl.4: C07D 403/04 , C07D 473/00 , //C07H19/067,C07H19/167, C07F7/18

(30) Priority: 16.05.88 US 194236

(43) Date of publication of application: 23.11.89 Bulletin 89/47

(84) Designated Contracting States: AT BE CH DE FR GB IT LI NL

(71) Applicant: F. HOFFMANN-LA ROCHE AG
Postfach 3255
CH-4002 Basel(CH)

(72) Inventor: Maehr, Hubert
30 Balsam Road
Wayne, N.J. 07470(US)

(74) Representative: Grossner, Lutz, Dr. et al
Grenzacher Strasse 124 Postfach 3255
CH-4002 Basel(CH)

## (54) Process for the preparation of 2',3'-dideoxynucleosides, and intermediates used therein.

(57) Herstellung von 2′.3′-Dideoxynucleosiden aus entsprechenden Nucleosiden unter Verwendung neuer Nucleosid-2′,3′-thiocarbonate.

EP 0 342 422 A2

# Verfahren zur Herstellung von 2′,3′-Dideoxynucleosiden und dabei verwendete neue Zwischenprodukte

Die vorliegende Erfindung betrifft ein Verfahren zur Herstellung von 2′,3′-Dideoxynucleosiden und dabei verwendete neue Zwischenprodukte.

Die Erfindung betrifft insbesondere ein Verfahren zur Herstellung von 2′,3′-Dideoxynucleosiden, dadurch gekennzeichnet, dass man

a) ein 5′-geschütztes Nucleosid mit Thiophosgen oder 1,1′-Thiocarbonyldiimidazol zum entsprechenden Nucleosid-2′,3′-thiocarbonat umsetzt,

b) das Thiocarbonat mit einem Reduktionsmittel zum entsprechenden Alken umsetzt,

c) das Alken zum entsprechenden Alkan hydriert und

d) die Schutzgruppen entfernt.

Der hier verwendete Ausdruck Nucleosid umfasst Uridin, Cytidin, Adenosin und Guanin. Der Ausdruck Schutzgruppe be deutet jede chemische Gruppe oder Rest, der allfällige unerwünschte Nebenreaktionen an potentiell reaktiven Stellen der Verbindung während des Syntheseverfahrens blockiert. Beispielsweise ist t-Butyldiphenylsilyl eine bekannte Schutzgruppe für die 5′-Hydroxygruppe von Nucleosiden; und aliphatische oder aromatische Acylgruppen sind beispielsweise Schutzgruppen für in Nucleosiden anwesende Aminogruppen. Niederalkyl bedeutet Alkylgruppen mit 1 bis 7 C-Atomen. Aryl bedeutet substituiertes oder unsubstituiertes Phenyl, wobei die Substituenten Halogen, Alkyl, Nitro oder Alkoxy sein können.

Wenn die 4-Aminogruppe von Cytosin oder die 6-Aminogruppe von Adenin geschützt ist, hat die geschützte Aminogruppe die Formel $-NHCOR^1$, worin $R^1$ Niederalkyl, Aryl oder substituirtes Aryl ist. Eine bevorzugte Aminoschutzgruppe ist Acetyl. Bevorzugte 5′-Hydroxyschutzgruppen sind t-Butyldiphenylsilyl, Pivaloyl oder Trityl, einschliesslich 4,4′-Dimethoxytriphenylmethyl und ähnlicher Tritylderivate.

Das bevorzugte Reduktionsmittel zur Umwandlung des Thiocarbonats in das entsprechende Alken ist 1,3-Dimethyl-2-phenyl-1,3-diazaphospholidin. Die Hydrierung des Alkens kann in Gegenwart eines Edelmetallkatalysators, wie Palladium auf Kohle durchgeführt werden. Die Entfernung der Schutzgruppe kann in an sich bekannter Weise erfolgen. Beispielsweise kann eine 5′-t-Butyldiphenylsilylgruppe durch Behandlung mit Tetrabutylammoniumfluorid und eine Acetylgruppe aus einer acetylierten Aminogruppe durch Behandlung mit Triäthylamin in Methanol entfernt werden.

In einer bevorzugten Ausführungsform betrifft die vorliegende Erfindung ein Verfahren zur Herstellung von 2′,3′-Dideoxycytidin welches dadurch gekennzeichnet ist, dass man

a) ein Cytidin, in dem die 4-Aminogruppe und die 5′-Hydroxygruppe geschützt sind, mit 1,1′-Thiocarbonyldiimidazol zum entsprechenden Nucleosid-2′,3′-thiocarbonat umsetzt,

b) das in Stufe a) erhaltene Produkt mit 1,3-Dimethyl-2-phenyl-1,3-diazaphospholidin zum entsprechenden 4,5′-geschützten 2′,3′-Didehydro-2′,3′-dideoxycytidin umsetzt,

c) das Produkt von Verfahrensstufe b) zum entsprechenden 4,5′-geschützten 2′,3′-Dideoxycytidin hydriert und

d) die Schutzgruppen aus dem in Stufe c) erhaltenen Produkt entfernt.

Das nachstehende Reaktionsschema erläutert eine bevorzugte Ausführungsform des erfindungsgemässen Verfahrens, wobei Ph Phenyl bedeutet.

FIGUR I

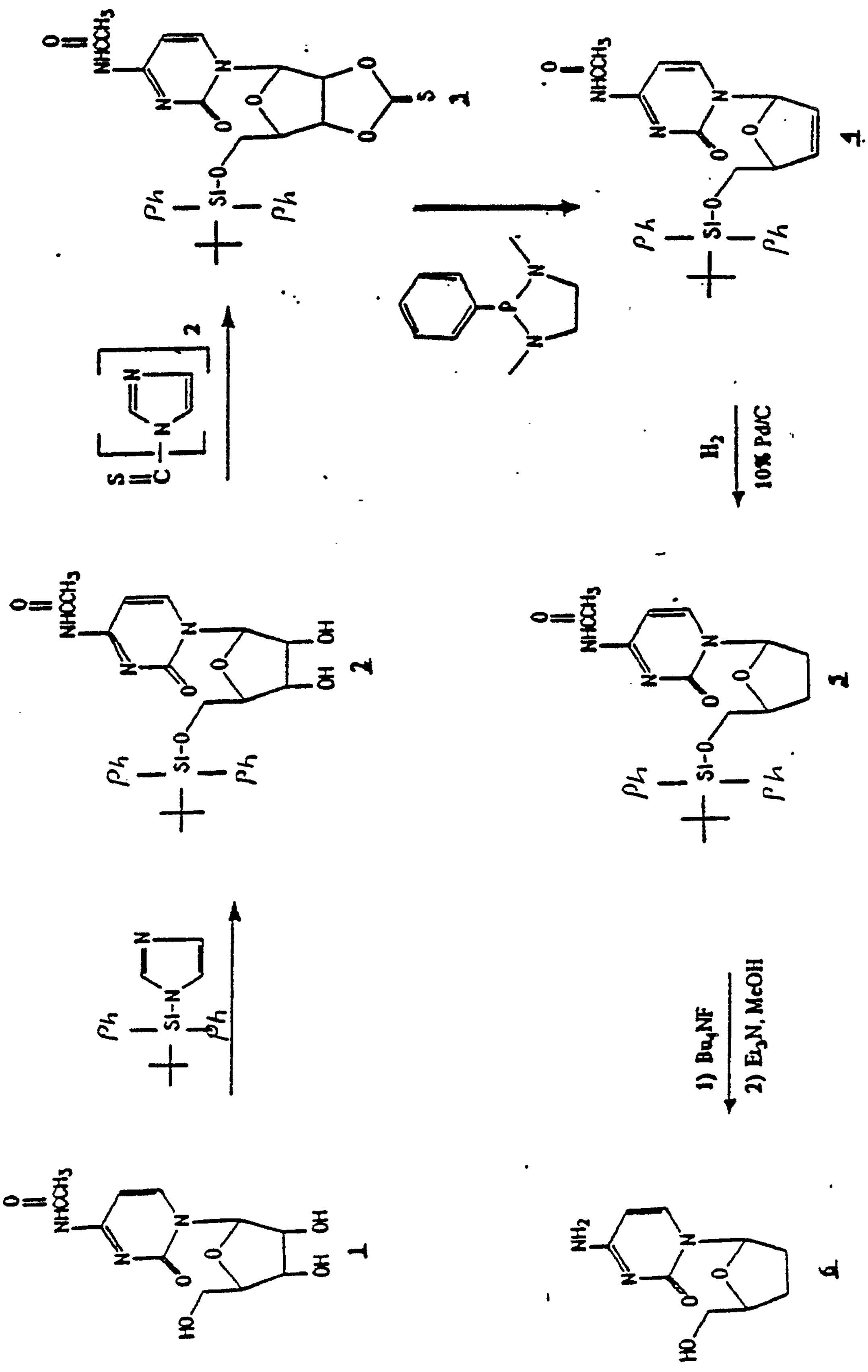

Die vorliegende Erfindung wird durch die nachstehenden Beispiele weiter erläutert.

Beispiel 1

Herstellung von N-Acetyl-5′-O-[(1,1-dimethyläthyl)-diphenylsilyl]cytidin (Verbindung 2 in Figur I)

2,33 g pulverisiertes N-Acetylcytidin wurden zu einer Lösung von t-Butyldiphenylsilylimidazol in DMF gegeben, die durch Zusatz von 1,36 g Imidazol unter Rühren zu einer Lösung von 1,37 g t-Butyldiphenylsilylchlorid in 10 ml über Molekularsieb getrockneten DMF hergestellt worden war. Die Suspension wurde unter Argonatmosphäre über Nacht bei Raumtemperatur gerührt, danach eingedampft und das Produkt durch Flash-Chromatographie mit Aethylacetat und dann 5% Methanol in Aethylacetat isoliert. Eindampfen lieferte 2,09 g der Verbindung 2als klebrigen Rückstand.

Beispiel 2

Herstellung von N-Acetyl-5′-O-[(1,1-dimethyläthyl)-diphenylsilyl]cytidin 2′,3′-thiocarbonat (3)

0,85 g 1,1′-Thiocarbonyldiimidazol wurden zu einer Lösung von 1,58 g 2 in 10 ml trockenem Dichlormethan gegeben. Die Lösung wurde 16 Stunden bei Raumtemperatur unter Argonatmosphäre gerührt. Das Produkt wurde durch Flash-Chromatographie mit Aethylacetat-Hexan (1:1 und dann 7:3) isoliert. Eindampfen lieferte 1,37 g 3 als farblosen Feststoff.

Beispiel 3

Herstellung von N-Acetyl-5′-O-[(1,1-dimethyläthyl)-diphenylsilyl]-2′,3′-didehydro-2′,3′-dideoxycytidin (4)

0,71 g des in Beispiel 2 erhaltenen Thiocarbonats 3 wurden in 8 ml trockenem Acetonitril gelöst und mit 0,4 ml 1,3-Dimethyl-2-phenyl-1,3-diazaphospholidin versetzt. Die Lösung wurde 7,5 Stunden unter Argonatmosphäre bei Raumtemperatur gerührt. Die erhaltene kristalline Suspension von 4 wurde auf ein kleines Volumen eingeengt und mit 5 ml Diäthyläther versetzt. Die Kristalle wurden abfiltriert und mit kleinen Portionen Diäthyläther gewaschen, wobei 0,303 g 4 erhalten wurden. Die Mutterlauge wurde eingedampft und mit Aethylacetat-Hexan (4:1) und danach mit Aethylacetat chromatographiert, wobei weitere 0,0887 g 4 als farblose Kristalle erhalten wurden. Insgesamt wurden 0,392 g 4, Schmelzpunkt 168-170° C erhalten.

Beispiel 4

Herstellung von N-Acetyl-5′-O-[(1,1-dimethyläthyl)-diphenylsilyl]-2′,3′-dideoxycytidin (5)

0,384 g des Alkens 4 wurden in 6 ml Methanol und 3 ml Tetrahydrofuran in Gegenwart von 200 ml 10% Pd/C mit einer Atmosphäre Wasserstoffdruck hydriert, bis die Wasserstoffaufnahme aufhörte (35 Minuten). Das Gemisch wurde über Celite abfiltriert und mit Methanol gewaschen. Die vereinigten Filtrate und Waschwässer wurden eingedampft und der erhaltene Rückstand in Acetonitril gelöst. Eine Spur unlösliches Material wurde abfiltriert und das Filtrat eingedampft, wobei 0,327 g 5 als farbloser Gummi erhalten wurden, der im nächsten Schritt ohne weitere Reinigung eingesetzt wurde.

Beispiel 5

Herstellung von 2′,3′-Dideoxycytidin (6)

Eine Lösung von 0,301 g 5 und 0,27 g Tetrabutylammoniumfluoridtrihydrat in Tetrahydrofuran wurde 3,5 Stunden bei Raumtemperatur gerührt. Eindampfen lieferte einen öligen Rückstand von $N^4$-Acetyl-2′,3′-dideoxycytidin. Der Rückstand wurde in 5 ml Methanol gelöst und mit 0,03 ml Triäthylamin versetzt. Die Lösung wurde 5 Stunden bei 47° C gehalten und dann zu einem Gemisch eines gummiartigen Materials und von Kristallen eingedampft. Dieses Gemisch wurde mit wenig Methanol und dann mit 8 ml Tetrahydrofuran versetzt. Die farblosen Kristalle wurden abfiltriert und mit Tetrahydrofuran gewaschen wobei 0,0776 g 6 erhalten wurden, das gemäss Reverse-Phasen-HPLC-Analyse (Waters µ-Bondapak C-18, 30 cm x 3,9 mm I.D., 15% Methanol-Wasser 1,3 ml/Minute, Nachweis bei 270 nm). Die Mutterlauge wurde eingedampft, der Rückstand in Wasser gelöst und über eine Säule von Amberlit IR 116

(Pyridiniumform) gegeben. Die Säule wurde mit Methanol gewaschen und mit 1M $NH_4OH$-Lösung eluiert. Die ammoniakalische Lösung wurde eingedampft, wobei weitere 0,0944 g 6 (93,4% rein) erhalten wurden. Die Gesamtausbeute betrug 0,172 g (95,6%). Schmelzpunkt des Produkts 210-211,5° C, $[\alpha]_D$ + 72,59° (c=0,53, Wasser).

## Claims

1. Verfahren zur Herstellung von 2',3'-Dideoxynucleosiden, dadurch gekennzeichnet, dass man

a) ein 5'-geschütztes Nucleosid mit Thiophosgen oder 1,1'-Thiocarbonyldiimidazol zum entsprechenden Nucleosid-2',3'-thiocarbonat umsetzt,

b) das Thiocarbonat mit einem Reduktionsmittel zum entsprechenden Alken umsetzt,

c) das Alken zum entsprechenden Alkan hydriert und

d) die Schutzgruppen entfernt.

2. Verfahren nach Anspruch 1, dadurch gekennzeichnet, dass das Nucleosid Uridin, Cytidin, Adenosin oder Geranin ist.

3. Verfahren nach Anspruch 1 oder 2, dadurch gekennzeichnet, dass das Reduktionsmittel 1,3-Dimethyl-2-phenyl-1,3-diazaphospholidin ist.

4. Verfahren nach Anspruch 1 zur Herstellung von 2',3'-Dideoxycytidin, dadurch gekennzeichnet, dass man

a) ein Cytidin, in dem die 4-Aminogruppe und die 5'-Hydroxygruppe geschützt sind, mit 1,1'-Thiocarbonyldiimidazol zum entsprechenden Nucleosid-2',3'-thiocarbonat umsetzt,

b) das in Stufe a) erhaltene Produkt mit 1,3-Dimethyl-2-phenyl-1,3-diazaphospholidin zum entsprechenden 4,5'-geschützten 2',3'-Didehydro-2',3'-dideoxycytidin umsetzt,

c) das Produkt von Verfahrensstufe b) zum entsprechenden 4,5'-geschützten 2',3'-Dideoxycytidin hydriert und

d) die Schutzgruppen aus dem in Stufe c) erhaltenen Produkt entfernt.

5. Verfahren nach Anspruch 4, dadurch gekennzeichnet, dass das geschützte Cytidin N-Acetyl-5'-O-[(1,1-dimethyläthyl)cytidin ist; die Hydrierung in Stufe c) in Gegenwart eines Palladiumkatalysators durchgeführt wird; und die Schutzgruppen durch Behandlung mit Tetrabutylammoniumfluorid bzw. Triäthylamin und Methanol entfernt werden.

6. Verbindungen der Formel

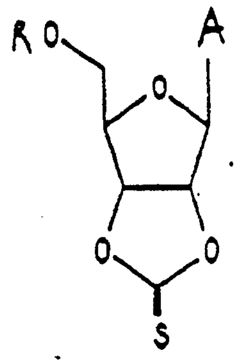

worin A ein Purinyl- oder Pyrimidinylrest und R eine Schutzgruppe ist.

7. Verbindungen gemäss Anspruch 6 worin A Cytosinyl oder Adeninyl ist, wobei die 4-Stellung des Cytosins und die 6-Stellung des Adenosins durch -NHCOR' substituiert sind, worin R' nieder-Alkyl, Aryl oder Halogen-, Alkyl-, Nitro- oder Alkoxy-substituiertes Aryl bedeutet.

8. Verbindungen gemäss Anspruch 7, worin R' Acetyl und R t-Butyldiphenylsilyl, Pivaloyl oder Trityl bedeuten.